(19) Europäisches Patentamt

European Patent Office

Office européen des brevets

(11) **EP 3 795 057 B1**

(12) **EUROPEAN PATENT SPECIFICATION**

(45) Date of publication and mention
of the grant of the patent:
**09.02.2022  Bulletin 2022/06**

(21) Application number: **18918770.1**

(22) Date of filing: **15.05.2018**

(51) International Patent Classification (IPC):
**B25J 9/16** *(2006.01)*    **A61B 1/005** *(2006.01)*

(52) Cooperative Patent Classification (CPC):
**A61B 1/0052; A61B 1/0051; B25J 9/16;**
A61B 1/00105; A61B 1/0055; A61B 1/0057;
A61B 1/008; A61B 2017/00327

(86) International application number:
**PCT/CN2018/086883**

(87) International publication number:
**WO 2019/218157 (21.11.2019 Gazette 2019/47)**

(54) **ENDOSCOPE STEERING ADJUSTMENT METHOD AND ENDOSCOPE**

VERFAHREN ZUR ENDOSKOPLENKEINSTELLUNG UND ENDOSKOP

PROCÉDÉ D'AJUSTEMENT DE DIRECTION D'ENDOSCOPE ET ENDOSCOPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**24.03.2021  Bulletin 2021/12**

(73) Proprietor: **Shenzhen Yateks Optical Electronic
Technology Co., Ltd.
Shenzhen, Guangdong 518172 (CN)**

(72) Inventor: **MA, Tianyue
Shenzhen, Guangdong 518172 (CN)**

(74) Representative: **Petculescu, Ana-Maria
Bayramoglu Law Offices LLC Türkiye
Irtibat Ofisi, Mira Office, Kanuni Sultan
Süleyman Boulevard, 5387. street
Beytepe, floor 12, no:50
06800 Cankaya, Ankara (TR)**

(56) References cited:
**EP-A1- 1 464 270        EP-A1- 3 159 124
CN-A- 1 684 625         CN-A- 101 622 107
CN-A- 104 470 450       CN-A- 105 407 785
CN-A- 106 102 645       CN-A- 106 413 619
US-B1- 6 493 608**

**Description**

**TECHNICAL FIELD**

Embodiments of the present disclosure relate to the technical field of detection, and in particular, relate to a method for adjusting a steering of an endoscope, and an endoscope.

**BACKGROUND**

Industrial endoscopes are video non-invasive detection instruments that combine electronic and optical technologies. Images of a confined space are captured by controlling the endoscope composed of a photoelectric conversion circuit and an LCD display, and the endoscope may enter various pipelines and equipment for real and efficient observation and image acquisition, to reflect use and damages inside the object to be inspected. Therefore, the endoscope is widely used in fields such as vehicle maintenance, foreign body observation in pipelines, and post-earthquake search and rescue.

In order to accommodate flexible steering operation in a narrow space, industrial endoscopes are often provided with one or a plurality of steering devices, each of which includes one or a plurality of steering joints. Each of the steering joints achieves the steering under traction of one or a plurality of steering steel wires. Each of the steering steel wires, under driving by a corresponding steering wheel, drives the steering joint to rotate in a corresponding direction, and an angle of the steering is generally proportional to a tension applied by the steering steel wire.

In the process of practicing the present disclosure, the inventor(s) have found that industrial endoscopes often need to have high protection abilities due to limitations of the working environment. Since industrial endoscopes may need to provide some construction and operation capabilities even in a narrow enclosed space, the steering steel wires or traction steel wires of the industrial endoscope need to bear a greater tension to perform corresponding operations. However, these steel wires may inevitably age after being stretched for multiple times, such that the industrial endoscope may fail to reach the destinations for operations.

A method according to the preamble of claim 1 is known from EP1464270.

**SUMMARY**

Embodiments of the present disclosure are mainly intended to provide a method for adjusting a steering of an endoscope, and an endoscope, to solve the technical problem that steering of the endoscope fails to be accurately controlled due to aging of steering steel wires.

In one aspect, embodiments of the present disclosure provide a technical solution of a method for adjusting a steering of an endoscope. The endoscope includes: a plurality of joints, a plurality of steering steel wires, and a plurality of drivers, wherein each of the steering steel wires passes through the plurality of joints, an elastic member is disposed between two adjacent joints of the joints, each of the steering steel wires is provided with a tension sensor, and one end of each of the steering steel wires is connected to a driver. The method includes: acquiring a bending direction and a bending angle $\theta_1$ of the endoscope when steering of the endoscope is to be adjusted; determining, based on the bending direction, a steering steel wire to be adjusted among the plurality of steering steel wires, and determining, based on the bending angle $\theta_1$, a tension value $F_{y1}$ of the steering steel wire to be adjusted and a rotation angle of the driver corresponding to the steering steel wire to be adjusted; controlling the driver corresponding to the steering steel wire to be adjusted to rotate by the rotation angle; reading a value of the tension sensor corresponding to the steering steel wire to be adjusted, and recording the value as $F_1$; identifying whether $F_1$ is less than $F_{y1}$; and controlling the driver corresponding to the steering steel wire to be adjusted to stop working when an absolute value of a difference between $F_1$ and $F_{y1}$ is less than a threshold, or continuing to control the driver corresponding to the steering steel wire to be adjusted to rotate in the same direction until the value of the tension sensor corresponding to the steering steel wire to be adjusted is equal to $F_{y1}$, if $F_1$ is less than $F_{y1}$.

Optionally, the method further includes: during rotation of the driver corresponding to the steering steel wire, acquiring in real time, by the tension sensor on a steering steel wire to be adjusted, a real-time tension withstood by the steering steel wire to be adjusted; identifying whether the real-time tension is greater than or equal to a predetermined tension threshold; controlling the driver corresponding to the steering steel wire to be adjusted to stop working if the real-time tension is greater than or equal to the predetermined tension threshold; and proceeding with the step of reading the value of the tension sensor corresponding to the steering steel wire to be adjusted, and recording the value as $F_1$ after the rotation angle of the driver corresponding to the steering steel wire to be adjusted is reached, if the real-time tension is not greater than or not equal to a predetermined tension threshold.

Optionally, the method further includes: outputting a prompt signal indicating that the endoscope fails to be bent to the bending angle when it is identified that the real-time tension is greater than or equal to the predetermined tension threshold.

Optionally, the method further includes: acquiring a difference between $F_1$ and $F_{y1}$ when $F_1$ is less than $F_{y1}$; identifying whether an absolute value of the difference between $F_1$ and $F_{y1}$ is greater than or equal to a predetermined aging threshold; outputting a prompt signal indicating that the steering steel wire to be adjusted severely ages if the absolute value of the difference between $F_1$ and $F_{y1}$ is greater than or equal to the predetermined aging threshold; and proceeding with the step of continuing to control the driver corresponding to the steering steel wire to be adjusted to rotate in the same direction until the value of the tension sensor corresponding to the steering steel wire to be adjusted is equal to $F_{y1}$, if the absolute value of the difference between $F_1$ and $F_{y1}$ is less than the predetermined aging threshold.

Optionally, the bending direction of the endoscope is in correspondence with the steering steel wire; and a bended angle of the endoscope, the tension of the steering steel wire, and the rotation angle of the driver are in corresponding with each other.

In another aspect, embodiments of the present disclosure provide a technical solution of an endoscope. The endoscope includes: a plurality of joints, a plurality of steering steel wires, a plurality of drivers, and a controller, wherein each of the steering steel wires passes through the plurality of joints, an elastic member is disposed between two adjacent joints of the joints, each of the steering steel wires is provided with a tension sensor, and one end of each of the steering steel wires is connected to a driver. The controller includes at least one processor and a memory communicably connected to the at least one processor. The memory stores instructions executable by the at least one processor. The instructions, when being executed by the at least one processor, cause the at least one processor to perform steps of: acquiring a bending direction and a bending angle $\theta_1$ of the endoscope when steering of the endoscope is to be adjusted; determining, based on the bending direction, a steering steel wire to be adjusted among the plurality of steering steel wires, and determining, based on the bending angle $\theta_1$, a tension value $F_{y1}$ of the steering steel wire to be adjusted and a rotation angle of the driver corresponding to the steering steel wire to be adjusted; controlling the driver corresponding to the steering steel wire to be adjusted to rotate by the rotation angle; reading a value of the tension sensor corresponding to the steering steel wire to be adjusted, and recording the value as $F_1$; identifying whether $F_1$ is less than $F_{y1}$; and controlling the driver corresponding to the steering steel wire to be adjusted to stop working when an absolute value of a difference between $F_1$ and $F_{y1}$ is less than a threshold, or continuing to control the driver corresponding to the steering steel wire to be adjusted to rotate in the same direction until the value of the tension sensor corresponding to the steering steel wire to be adjusted is equal to $F_{y1}$, if $F_1$ is less than $F_{y1}$.

Optionally, the instructions, when being executed by the at least one processor, cause the at least one processor to perform steps of: during rotation of the driver corresponding to the steering steel wire, acquiring in real time, by the tension sensor on a steering steel wire to be adjusted, a real-time tension withstood by the steering steel wire to be adjusted; identifying whether the real-time tension is greater than or equal to a predetermined tension threshold; controlling the driver corresponding to the steering steel wire to be adjusted to stop working if the real-time tension is greater than or equal to the predetermined tension threshold; and proceeding with the step of reading the value of the tension sensor corresponding to the steering steel wire to be adjusted, and recording the value as Fi after the rotation angle of the driver corresponding to the steering steel wire to be adjusted is reached, if the real-time tension is not greater than or not equal to a predetermined tension threshold.

Optionally, the instructions, when being executed by the at least one processor, cause the at least one processor to perform a step of: outputting a prompt signal indicating that the endoscope fails to be bent to the bending angle when it is identified that the real-time tension is greater than or equal to the predetermined tension threshold.

Optionally, the instructions, when being executed by the at least one processor, cause the at least one processor to perform steps of: acquiring a difference between $F_1$ and $F_{y1}$ when $F_1$ is less than $F_{y1}$; identifying whether an absolute value of the difference between $F_1$ and $F_{y1}$ is greater than or equal to a predetermined aging threshold; outputting a prompt signal indicating that the steering steel wire to be adjusted severely ages if the absolute value of the difference between $F_1$ and $F_{y1}$ is greater than or equal to the predetermined aging threshold; and proceeding with the step of continuing to control the driver corresponding to the steering steel wire to be adjusted to rotate in the same direction until the value of the tension sensor corresponding to the steering steel wire to be adjusted is equal to $F_{y1}$, if the absolute value of the difference between $F_1$ and $F_{y1}$ is less than the predetermined aging threshold.

Optionally, the bending direction of the endoscope is in correspondence with the steering steel wire; and a bended angle of the endoscope, the tension of the steering steel wire, and the rotation angle of the driver are in corresponding with each other.

As compared with the related art, the embodiments of the present disclosure achieve the following beneficial effects: When steering of the endoscope is to be adjusted, a tension sensor is disposed on the steering steel wire, and a value of the tension sensor is acquired, such that a tension Fi of the steering steel wire is acquired. If the tension $F_1$ is less than $F_{y1}$ corresponding to a bending angle of the endoscope, the driver is controlled to stop working when an absolute value of a difference between $F_1$ and $F_{y1}$ is less than a threshold, or the driver is controlled to pull back the steel wire until the value of the tension sensor corresponding to the steel wire to be adjusted is equal to $F_{y1}$, such that the bended angle of the endoscope reaches a predetermined bending angle. In this way, the case where steering of the endoscope fails to be accurately controlled due to aging of the steering steel wire is avoided.

**BRIEF DESCRIPTION OF THE DRAWINGS**

For clearer descriptions of the technical solutions according to the embodiments of the present disclosure, hereinafter brief description is given with reference to the accompanying drawings for illustrating the embodiments. Apparently, the accompanying drawings described hereinafter only illustrate some embodiments of the present disclosure, and other accompanying drawings may also be derived based on these accompanying drawings.

FIG. 1 is a schematic structural view of an endoscope according to an embodiment of the present disclosure;
FIG. 2 is a schematic diagram of modules of the endoscope according to an embodiment of the present disclosure;
FIG. 3 is a schematic flowchart of a method for adjusting a steering of the endoscope according to an embodiment of the present disclosure;
FIG. 4 is a schematic flowchart of another method for adjusting a steering of the endoscope according to an embodiment of the present disclosure; and
FIG. 5 is a schematic flowchart of still another method for adjusting a steering of the endoscope according to an embodiment of the present disclosure.

Reference numerals and denotations thereof: 101-endoscope, 10-steering joint, 101-first joint, 102-second joint, 103-third joint, 20-steering steel wire, 30-elastic member, 40-tension sensor, 50-driver, 60-controller, 70-memory, 80-processor.

**DETAILED DESCRIPTION**

For better understanding of the present disclosure, the present disclosure is described in detail with reference to attached drawings and specific embodiments. It should be noted that, when an element is defined as "being secured or fixed to" another element, the element may be directly positioned on the element or one or more centered elements may be present therebetween. When an element is defined as "being connected or coupled to" another element, the element may be directly connected or coupled to the element or one or more centered elements may be present therebetween. As used herein, the terms "vertical," "horizontal," "left," "right," "inner," "outer," and similar expressions are only for illustration purposes.

Unless the context clearly requires otherwise, throughout the specification and the claims, technical and scientific terms used herein denote the meaning as commonly understood by a person skilled in the art. Additionally, the terms used in the specification of the present disclosure are merely for description the embodiments of the present disclosure, but are not intended to limit the present disclosure. As used herein, the term "and/or" in reference to a list of two or more items covers all of the following interpretations of the term: any of the items in the list, all of the items in the list and any combination of the items in the list.

Specifically, the embodiments of the present disclosure are described in detail with reference to a method for adjusting a steering of an endoscope and an endoscope in combination with the accompanying drawings.

Generally, a bended angle of the endoscope is adjusted by a steel wire. However, since the steel wire may age over time, once the steel wire ages, the bended angle of the endoscope fails to be accurately adjusted based on the original rotation angle of a driver. If the bended angle of the endoscope is adjusted by compensating a tension for the steel wire, a tension to be compensated for the steel wire may not be determined because aging of the steel wire fails to be determined.

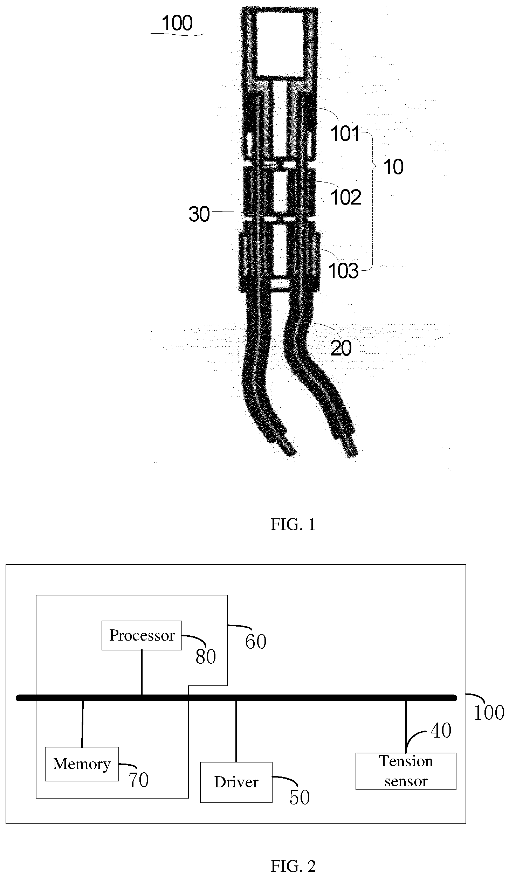

FIG. 1 is a schematic structural view of an endoscope according to an embodiment of the present disclosure. FIG. 2 is a schematic diagram of modules of the endoscope according to an embodiment of the present disclosure.

As illustrated in FIG. 1 and FIG. 2, an endoscope 100 includes a plurality of joints 10, a plurality of steering steel wires 20, a plurality of drivers 50, and a controller 60. Each of the steering steel wires 20 passes through the plurality of joints 10, and an elastic member 30 is disposed between two adjacent joints of the joints 10. In some embodiments, the plurality of joints 10 may include a first joint 101, a second joint 102, and a third joint 103, and the elastic member 30 is connected between the first joint 101 and the second joint 102, and between the second joint 102 and the third joint 103 respectively. In this embodiment, the elastic member 30 employs a spring, and the plurality of steering steel wires 20 all pass through the first joint 101, the second joint 102, and the third joint 103.

Each of the steering steel wires 20 is provided with a tension sensor 40. The tension sensor 40 is configured to monitor in real time tension of the steering steel wire 20. One end of each of the steering steel wires 20 is connected to a driver 50. The driver 50 is configured to adjust a bended angle of the endoscope 100 by pulling back or releasing the steering steel wire 20, the controller 60 is configured to receive the tension of the steering steel wire 20 fed back by the tension sensor 40, and configured to control start, stop and a rotation angle of the driver 50. In some embodiments, the controller 60 may drive, based on feedback tension of the steering steel wire 20, the corresponding driver 50 to pull back or release the steering steel wire 20, such that the endoscope 100 is bent by a predetermined angle towards a predetermined direction. In this embodiment, the driver 50 may employ a step motor, and the tension sensor 40 may be a strain gauge or an optical fiber gate sensor.

The controller 60 includes at least one processor 80, and a memory 70 communicably connected to the at least one processor 80. The at least one processor 80 is connected to the driver 50 and the tension sensor 40 respectively. The memory 70 stores instructions executable by the at least one processor 80, and the instructions are executed by the at least one processor 80.

FIG. 2 uses one processor 80 as an example.

The processor 80 and the memory 70 may be connected via a bus or in another manner, and FIG. 2 uses the bus as an example.

The memory 70, as a non-volatile computer-readable storage medium, may be configured to store non-volatile software programs, non-volatile computer-executable programs and modules, for example, the program instructions/modules corresponding to a method for adjusting a steering of an endoscope according to an embodiment of the present disclosure. The non-volatile software programs, instructions and modules stored in the memory 70, when being executed, cause the at least one processor 80 to perform various function applications and data processing of the endoscope, that is, performing a method for adjusting a steering of an endoscope according to an embodiment of the present disclosure hereinafter.

The memory 70 may include a program memory area and a data memory area, wherein the program memory area may store operation systems and application programs needed by at least one function; and the data memory area may store data created according to the usage of the endoscope. In addition, the memory 70 may include a high-speed random access memory, or include a non-volatile memory, for example, at least one disk storage device, a flash memory device, or another non-volatile solid storage device. In some embodiments, the memory 70 optionally includes a memory 70 remotely arranged relative to the processor 80, and such remote memory 70 may be connected to the endoscope over the network. Examples of the above network include, but not limited to, the Internet, Intranet, local area network, mobile communication network and a combination thereof.

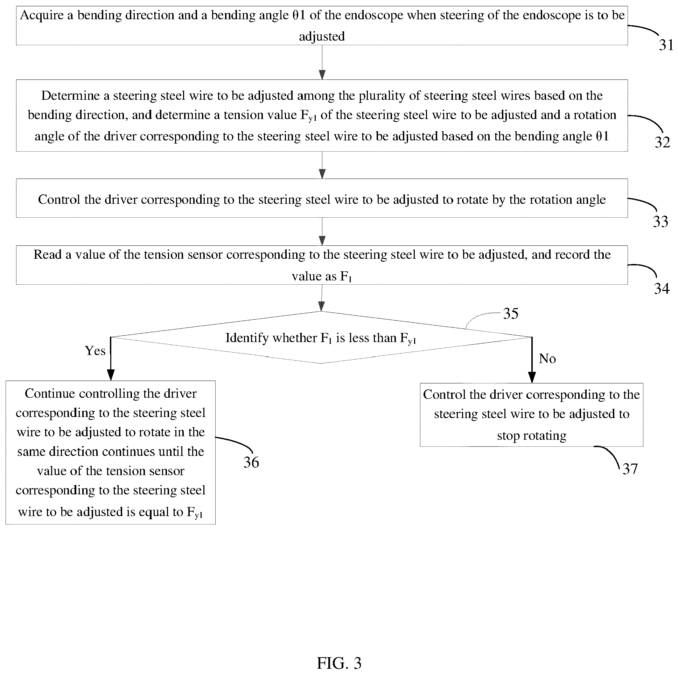

One or a plurality of modules are stored in the memory 70, and when executed by the one or a plurality of processors 80, perform a method of for adjusting a steering of an endoscope according to any of method embodiments hereinafter, for example, performing steps 31 to 54 in the methods as illustrated in FIG. 3 to FIG. 5.

An embodiment of the present disclosure further provides a non-volatile computer-readable storage medium. The non-volatile computer-readable storage medium stores computer-executable instructions, which, when executed by the one or a plurality of processors 80, for example, the processor 80 as illustrated in FIG. 2, may cause the one or a plurality of processors 80 to perform a method for adjusting a steering of an endoscope according to any of method embodiments hereinafter, for example, performing steps 31 to 54 in the methods as illustrated in FIG. 3 to FIG. 5.

In an embodiment of the present disclosure, when steering of an endoscope is to be adjusted, a tension sensor is disposed on the steering steel wire, and a value of the tension sensor is acquired, such that a tension $F_1$ of the steering steel wire is acquired. If the tension $F_1$ is less than $F_{y1}$ corresponding to a bending angle of the endoscope, the driver is controlled to pull back the steering steel wire until the value of the tension sensor corresponding to the steering steel wire to be adjusted is equal to $F_{y1}$, such that a bended angle of the endoscope reaches a predetermined bending angle. In this way, the case where steering of the endoscope fails to be accurately controlled due to aging of the steering steel wire is avoided.

A first embodiment of the present disclosure provides a method for adjusting a steering of an endoscope, which is applicable to the endoscope 100 as described above. The method includes the following steps:

In step 31, a bending direction and a bending angle $\theta_1$ of the endoscope are acquired when steering of the endoscope is to be adjusted.

In this embodiment, during working of the endoscope, steering of the endoscope needs to be adjusted in real time to observe a region to be detected. When steering of the endoscope needs to be adjusted, steering may be adjusted based on the bending direction of the endoscope. For example, in this case, a bended direction of the endoscope is on the left side of the region to be detected, the bending direction of the endoscope is deflected towards the right side, and the endoscope may be farther away from the region to be detected and adjustment may be more difficulty if the bending direction is further deflected towards the left side. Similarly, if a bended direction of the endoscope is on the right side of the region to be detected in this case, the bending direction of the endoscope is deflected towards the left side, and the difficulty in adjustment may be increased if the bending direction is further deflected towards the right side. Therefore, during steering adjustment of the endoscope, the bended direction of the endoscope needs to be acquired first, and then steering of the endoscope is adjusted based on the bending direction of the endoscope. In this way, time elapsed for adjusting the bending angle of the endoscope may be shortened, and working efficiency of the endoscope may be improved.

It may be understood that the bending angle of the endoscope may also be a direction in which the endoscope is adjusted to the bending angle $\theta_1$. For example, if the region to be detected of the endoscope needs to be adjusted by an angle $\theta_1$, it may be understood that the endoscope needs to be adjusted to the left side by $\theta_1$ degree, or adjusted to the right side by $\theta_1$ degree. Therefore, the bending direction of the endoscope needs to be first determined to accurately determine the bending angle of the endoscope.

Nevertheless, the bending angle of the endoscope still fails to be accurately adjusted when the bending direction is determined whereas the bending angle is not determined. The bending angle $\theta_1$ of the endoscope also needs to be determined. After the bending direction of the endoscope is determined, the bending angle of the endoscope is adjusted only by adjusting a deflection angle of the endoscope to $\theta_1$.

In step 32, a steering steel wire to be adjusted is determined among the plurality of steering steel wires based on the bending direction, and a tension value $F_{y1}$ of the steering steel wire to be adjusted and a rotation angle of the driver corresponding to the steering steel wire to be adjusted are determined based on the bending angle $\theta_1$.

If the endoscope needs to be adjusted to be bent to the right by $\theta_1$, the steering steel wire on the right of the endoscope only needs to be adjusted, with no need to adjust the steering steel wire on the left of the endoscope. That is, if the endoscope before adjustment is bent to the left, the steering steel wires connecting to the endoscope are in such a state that the steering steel wire on the right of the endoscope is longer than the steering steel wire on the left of the endoscope. If the endoscope needs to be adjusted to the right, the length of the steering steel wire on the right side of the endoscope needs to be shortened. Generally, the endoscope is bent to the right by $\theta_1$ by pulling back the steering steel wire on the right of the endoscope by the driver. Therefore, the steering steel wire to be adjusted is determined based on the bending direction of the endoscope, which prevents increase of difficulty in adjusting the endoscope due to mis-adjustment of the steering steel wire.

It may be understood that after the steering steel wire ages, the length of the steering steel wire is increased, and during adjustment of the steering steel wire, a friction force $F_m$ may be generated between the steering steel wire and a tubular wall of the endoscope. In this case, the driver needs to provide a traction of:

$$F_q = F_m + m * \text{front-end bending angle } \theta_1 \text{ of the endoscope}$$

wherein m is a constant;

> $F_m$ is the friction force between the steering steel wire and the tubular wall of the endoscope; and
> $F_q$ is the traction applied by the driver during adjustment of the endoscope.

When the steering steel wire ages, the length of the steering steel wire may be increased. Assuming that the length of the steering steel wire before the steering steel wire ages is L, then after aging, the length of the steering steel wire is L'. After the steering steel wire ages, the bended angle of the endoscope may deviate from the region to be detected. To restore the endoscope to its original state, the deflection angle of the endoscope is corrected by adjusting the steering steel wire.

The driver is controlled to pull back the steering steel wire to be adjusted. When the tensile amount of the steering steel wire is in the range of [0, L'-L], the value of the tension sensor on the steering steel wire is only a dynamic friction force $F_m$. The process of adjusting the steering steel wire is as follows:

First, the endoscope is restored to the natural state, and then the driver is controlled to automatically rotate to pull back the steering steel wire. The driver is stopped until the value of the tension sensor $F = F_m + p$, wherein p is a predetermined lower threshold and is 0.1 N in this embodiment. In this case, the rotation angle of the driver is recorded as $A_1$ (In this case, the relaxation section of the steel wire due to aging is rightly stretched straight. However, the bended angle of the endoscope is $\theta_1 = 0$).

The driver is continuously controlled to pull back the steering steel wire, and the driver is stopped until the value of the tension sensor $F = F_m + q$, wherein q is a predetermined upper threshold, and is a tension obtained when the endoscope is bent to a maximum angle before delivery from factory. In this embodiment, q is 5 N. In this case, the rotation angle of the steer is recorded as $A_2$ (in this case, the bended angle of the endoscope has reached the maximum angle $\theta_2$).

As known from the above, the bending direction of the endoscope is in correspondence with the steering steel wire; and the bended angle of the endoscope, the tension of the steering steel wire, and the rotation angle of the driver are in correspondence with each other.

Before delivery from factory of the endoscope, the bended angle of the endoscope, the tension of the steering steel wire, the rotation angle of the driver all correspond to specified standard values. These standard values form a standard library. The rotation angle of the corresponding driver and the tension $F_{y1}$ of the steel wire to be adjusted may be searched out in the standard library based on the bending angle $\theta_1$ of the endoscope. Then the bending angle $\theta_1$ of the endoscope may be adjusted based on the rotation angle of the driver.

In step 33, the driver corresponding to the steering steel wire to be adjusted is controlled to rotate by the rotation angle.

Based on the rotation angle of the corresponding driver that is searched out from the standard library during adjustment of the bending angle $\theta_1$ of the endoscope, if the bending angle of the endoscope is $\theta_1$, the rotation angle of the driver is $A_1$. In this case, the driver is controlled to pull back the steering steel wire. When the rotation angle of the driver reaches $A_1$, the driver is stopped.

Theoretically, when the rotation angle of the driver reaches $A_1$, the bended angle of the endoscope is rightly adjusted to $\theta_1$, and the tension of the corresponding steering steel wire is equal to $F_{y1}$.

In step 34, a value of the tension sensor corresponding to the steering steel wire to be adjusted is read, and the value is recorded as $F_1$.

When the driver is stopped from pulling back the steering steel wire, the value of the tension sensor corresponding to the steering steel wire is read and recorded as $F_1$. It should be noted that $F_1$ is a traction applied by the driver to the steering steel wire and is monitored in real time, that is, the value of the tension sensor corresponding to the steering steel wire, but not the corresponding $F_{y1}$ searched out from the standard library when the bending angle of the endoscope is $\theta_1$.

In step 35, whether $F_1$ is less than $F_{y1}$ is identified.

The tension $F_1$ of the corresponding tension sensor during pulling back the steering steel wire by the driver when the bended angle of the endoscope is adjusted to $\theta_1$ is compared with the tension $F_{y1}$ of the corresponding steering steel wire that is searched out from the standard library when the bended angle of the endoscope is $\theta_1$.

In step 36, the driver corresponding to the steering steel wire to be adjusted is controlled to rotate in the same direction continues until the value of the tension sensor corresponding to the steering steel wire to be adjusted is equal to $F_{y1}$, if $F_1$ is less than $F_{y1}$.

If $F_1$ is less than $F_{y1}$, the bended angle of the endoscope does not reach $\theta_1$, and the driver needs to be continuously controlled to pull back the steering steel wire until the corresponding tension $F_1$ of the steering steel wire is equal to $F_{y1}$. In this case, the bended angle of the endoscope reaches $\theta_1$.

In step 37, the driver corresponding to the steering steel wire to be adjusted to is controlled to stop rotating, if $F_1$ is not less than $F_{y1}$.

If $F_1$ is equal to $F_{y1}$, it may be determined that the steering steel wire does not age, and the bended angle of the endoscope reaches $\theta_1$. In this case, the steering steel wire does not need to be adjusted, that is, the driver is controlled to stop rotating. However, it is impossible that $F_1$ is greater than $F_{y1}$, since when the steering steel wire ages, the steering steel wire is relaxed, and the rotation angle when the driver applies a traction to the steering steel wire is greater than the rotation angle of the driver when the steering steel wire does not age. Therefore, the driver applies different tensions for pulling back the steering steel wire to the same angle, and the tension received when the steering steel wire ages is apparently less than the tension received when the steering steel wire does not age.

The embodiment of the present disclosure achieves the following beneficial effects: When steering of the endoscope is to be adjusted, a tension sensor is disposed on the steering steel wire, and a value of the tension sensor is acquired, such that a tension $F_1$ of the steering steel wire is acquired. If the tension $F_1$ is less than $F_{y1}$ corresponding to a bending angle of the endoscope, the driver is controlled to pull back the steel wire until the value of the tension sensor corresponding to the steering steel wire to be adjusted is equal to $F_{y1}$, such that the bended angle of the endoscope reaches a predetermined bending angle. In this way, the case where steering of the endoscope fails to be accurately controlled due to aging of the steel wire is avoided.

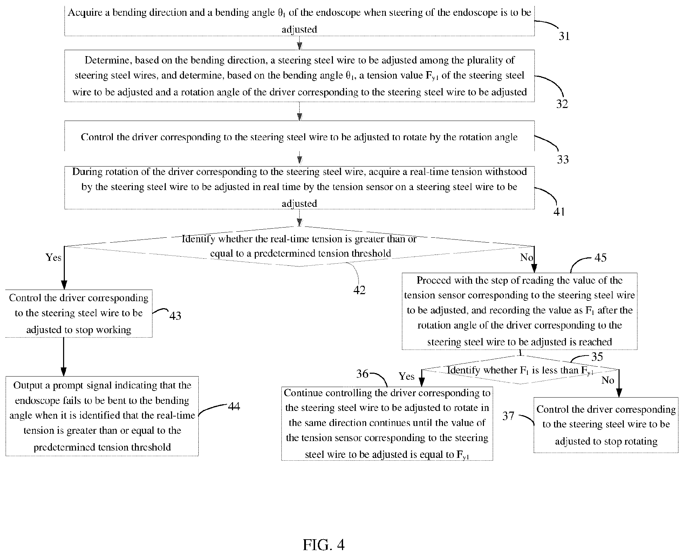

As illustrated in FIG. 4, a second embodiment of the present disclosure provides another method for adjusting a steering of an endoscope. Different from the method as described in the above embodiment, the method further includes the following steps:

In step 41, during rotation of the driver corresponding to the steering steel wire, a real-time tension withstood by the steering steel wire to be adjusted is acquired in real time by the tension sensor on a steering steel wire to be adjusted.

After the steering steel wire to be adjusted is determined, the driver is controlled to pull back the steering steel wire to be adjusted. During pulling back the steering steel wire, the steering steel wire withstands the traction applied by the controller, and the tension of the tension sensor is acquired in real time by monitoring the tension sensor on the steering steel wire. The tension indicated by the tension sensor is a real-time tension withstood by the steering steel wire to be adjusted.

In step 42, whether the real-time tension is greater than or equal to a predetermined tension threshold is identified.

The predetermined tension threshold is predefined artificially according to adjustment experience. The predetermined tension threshold is also a maximum tension withstood by the steering steel wire. Whether the bended angle of the endoscope may be adjusted by pulling back the steering steel wire by the driver may be identified based on whether the real-time tension withstood by the steering steel wire is greater than or equal to the predetermined tension.

In step 43, the driver corresponding to the steering steel wire to be adjusted is controlled to stop working if the real-time tension is greater than or equal to the predetermined tension threshold.

If the tension acquired from the tension sensor corresponding to the steering steel wire is greater than or equal to the predetermined tension threshold, the tension applied to the steering steel wire has reached or exceeded the maximum tension withstood by the steering steel wire. In this case, if the driver is continuously controlled to pull back the steering steel wire, the steering steel wire may break. Therefore, for prevention more severe aging of the steering steel wire, the driver corresponding to the steering steel wire needs to be immediately controlled to stop pulling back the steering steel wire.

In step 44, a prompt signal indicating that the endoscope fails to be bent to the bending angle is output when it is identified that the real-time tension is greater than or equal to the predetermined tension threshold.

The prompt signal may be "Warning," "No Working," or "Wrong" displayed on the screen of the endoscope. For example, during working of the driver, when the screen of the endoscope displays "Warning," the real-time tension of the steering steel wire is greater than or equal to the predetermined tension threshold, the tension withstood by the steering steel wire reaches the maximum value, and the driver may fail to be controlled to pull back the steering steel wire to further adjust the bended angle of the endoscope, that is, the endoscope fails to be bent to the bending angle.

In step 45, the process proceeds with the step of reading the value of the tension sensor corresponding to the steering steel wire to be adjusted, and recording the value as $F_1$, after the rotation angle of the driver corresponding to the steering steel wire to be adjusted is reached, if the real-time tension is not greater than or not equal to a predetermined tension threshold.

If the tension acquired from the tension sensor corresponding to the steering steel wire is less than the predetermined tension threshold, the driver may pull back the steering steel wire to be adjusted to adjust the bended angle of the endoscope, the driver is controlled to rotate to the rotation angle based on the rotation angle of the driver corresponding to the steering steel wire to be adjusted, and the value of the tension sensor corresponding to the steering steel wire to be adjusted is acquired and recorded as $F_1$.

If $F_1$ is less than $F_{y1}$, the driver is continuously controlled to pull back the steering steel wire in the same direction, until the value of the tension sensor corresponding to the steering steel wire to be adjusted is equal to $F_{y1}$. In this case, the bended angle of the endoscope reaches the predetermined bending angle $\theta_1$.

This embodiment achieves the following beneficial effect: When steering of the endoscope needs to be adjusted, a tension withstood by the steering steel wire that is acquired in real time is compared with a predetermined tension threshold, and whether the real-time tension is greater than or equal to the predetermined tension threshold is identified; if the real-time tension is greater than or equal to the predetermined tension threshold, a driver corresponding to the steering steel wire to be adjusted is controlled to stop working, and otherwise, the driver corresponding to the steering steel wire is continuously controlled to rotate. In this case, breakage of the steering steel wire due to over-great tension applied to the steering steel wire is prevented, and adjustment efficiency of the bended angle of the endoscope is improved.

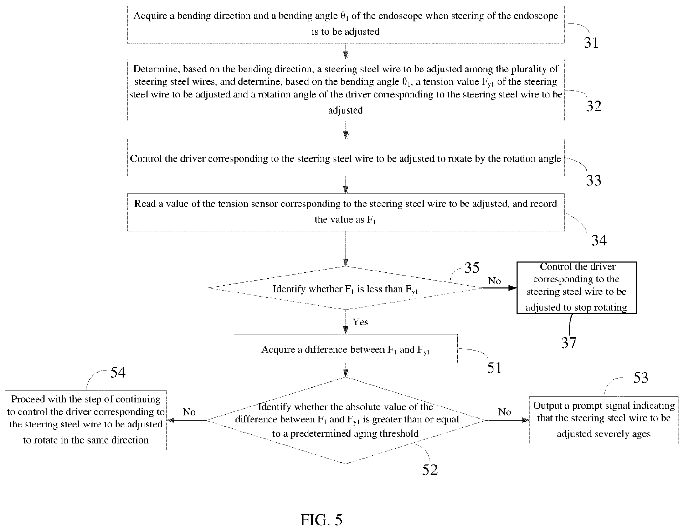

As illustrated in FIG. 5, a third embodiment of the present disclosure provides another method for adjusting a steering of an endoscope. The method further includes the following steps:

In step 31, a bending direction and a bending angle $\theta_1$ of the endoscope are acquired when steering of the endoscope is to be adjusted.

In step 32, a steering steel wire to be adjusted among the plurality of steering steel wires is determined based on the bending direction, and a tension value $F_{y1}$ of the steering steel wire to be adjusted and a rotation angle of the driver corresponding to the steering steel wire to be adjusted are determined based on the bending angle $\theta_1$.

In step 33, the driver corresponding to the steering steel wire to be adjusted is controlled to rotate by the rotation angle.

In step 34, a value of the tension sensor corresponding to the steering steel wire to be adjusted is read, and the value is recorded as $F_1$.

In step 35, whether $F_1$ is less than $F_{y1}$ is identified. Step 37 is performed if $F_1$ is not less than $F_{y1}$; and otherwise, step 51 is performed.

In step 37, the driver corresponding to the steering steel wire to be adjusted to is controlled to stop rotating, if $F_1$ is not less than $F_{y1}$.

In step 51, a difference between $F_1$ and $F_{y1}$ is acquired if $F_1$ is less than $F_{y1}$;

Step 35 is continued, and the difference between $F_1$ and $F_{y1}$ is acquired if $F_1$ is less than $F_{y1}$, and is recorded as $\triangle F$.

It should be understood that if $F_1$ is less than $F_{y1}$, whether an absolute value of the difference between $F_1$ and $F_{y1}$ is less than a threshold is identified. If the absolute value of the difference is less than the threshold, step 52 is performed.

System and working condition may be subject to fluctuations. During working of the endoscope, the fluctuations may affect adjustment of the deflection angle of the endoscope. A threshold is defined to filter impacts caused by minor fluctuations. In this embodiment, the threshold is defined to 0.2 N.

For example, if $F_1$ is 3.2 N and $F_{y1}$ is 3.8 N, the difference between $F_1$ and $F_{y1}$ is 0.6 N. In this case, the difference between $F_1$ and $F_{y1}$ is greater than the threshold 0.2 N, and the driver is continuously controlled to pull back the steering steel wire such that the tension of the steering steel wire reaches $F_{y1}$.

If $F_1$ is less than $F_{y1}$, whether the absolute value of the difference between $F_1$ and $F_{y1}$ is less than the threshold is identified. If the absolute value of the difference is less than the threshold, the driver is controlled to stop pulling back of the steering steel wire.

For example, if $F_1$ is 3.5 N, $F_{y1}$ is 3.6 N, the difference between $F_1$ and $F_{y1}$ is 0.1 N, which is less than the threshold 0.2 N. The difference between $F_1$ and $F_{y1}$ is caused by the fluctuations of the system and the working conditions. In this case, the difference is within an error range of the system, and the deflection angle of the endoscope does not need to be adjusted. The deflection angle does not affect working of the endoscope. If a tension is further applied to the steering steel wire, a further load may be increased for the steering steel wire, which may even accelerate aging of the steering steel wire, and meanwhile increases workload of the working stuff.

In step 52, whether the absolute value of the difference between $F_1$ and $F_{y1}$ is greater than or equal to a predetermined aging threshold is identified.

The predetermined aging threshold may be understood as aging degree of the steering steel wire. If an absolute value of the difference between $F_1$ and $F_{y1}$ is great, the aging degree of the steering steel wire is great. If the absolute value of the difference between $F_1$ and $F_{y1}$ is small, the aging degree of the steering steel wire is small. In case of aging, not all the steering steel wires may be unlimitedly applied with a tension to adjust the bended angle of the endoscope. If a steering steel wire severely ages, when a tension applied to the steering steel wire reaches to a specific value, the steering steel wire may break, and as a result the endoscope collapses. According to experience in adjusting the endoscope, the predetermined aging threshold may be defined. The aging degree of the steering steel wire may be determined according to a relationship between the absolute value of the difference between $F_1$ and $F_{y1}$ and the predetermined aging threshold, and whether a tension is to be applied to the steering steel wire to adjust the bended angle of the endoscope is identified.

In step 53, a prompt signal indicating that the steering steel wire to be adjusted severely ages is output if the absolute value of the difference between $F_1$ and $F_{y1}$ is greater than or equal to the predetermined aging threshold.

If the absolute value of the difference between $F_1$ and $F_{y1}$ is greater than or equal to the predetermined aging threshold, it may be determined that the steering steel wire severely ages, and the bended angle of the endoscope may not be further adjusted by the steering steel wire. If the bended angle is further adjusted by the steering steel wire, the steering steel wire may break. In this case, the only solution is to replace the steering steel wire to prolong life time of the endoscope.

When the absolute value of the difference between $F_1$ and $F_{y1}$ is greater than or equal to the predetermined aging threshold, the information is transferred to a processor. The processor outputs the prompt signal indicating that the steering steel wire to be adjusted severely ages. This signal may prompt the working stuff to replace the steering steel wire.

In step 54, the process proceeds with the step of continuing to control the driver corresponding to the steering steel wire to be adjusted to rotate in the same direction, if the absolute value of the difference between $F_1$ and $F_{y1}$ is less than the predetermined aging threshold.

The term "less than" means that if the absolute value of the difference between $F_1$ and $F_{y1}$ is less than the predetermined aging threshold, it may be determined that the steering steel wire slightly ages, and the steering steel wire may be further adjusted to acquire the difference between $F_1$ and $F_{y1}$. The driver is controlled to continuously pull back the steering steel wire along the same direction, such that an increment of the value of the tension sensor corresponding to the steering steel wire is equal to the difference between $F_1$ and $F_{y1}$, or the value of the tension sensor corresponding to the steering steel wire is increased to $F_{y1}$. The aging degree of the steering steel wire is determined based on the predetermined aging threshold. In this way, the steering steel wire is prevented from breaking due to an over-great tension applied to the steering steel wire.

The embodiments of the present disclosure achieve the following beneficial effects: When the bended angle of the endoscope needs to be adjusted, a tension of the steering steel wire is acquired in real time by the tension sensor and hence the absolute value of the difference between the real-time tension $F_1$ and $F_{y1}$ is acquired, whether the absolute value of the difference between the real-time tension $F_1$ and $F_{y1}$ is greater than or equal to the predetermined aging threshold is identified; if the absolute value is greater than or equal to the predetermined aging threshold, it is determined that the steering steel wire to be adjusted slightly ages, if not, the steering steel wire slightly ages, and the steering steel wire may be further adjusted until the tension of the steering steel wire reaches $F_{y1}$. By this method, the aging degree of the steering steel wire may be determined, and the steering steel wire may be correspondingly adjusted based on the aging degree of the steering steel wire. In one aspect, the steering steel wire may be prevented from breaking due to an over-great tension applied thereon, and life time of the steering steel wire may be prolonged. In another aspect, workload of the working stuff may be reduced, and working efficiency of the endoscope may be improved.

Described above are exemplary embodiments of the present disclosure, but are not intended to limit the scope of the present disclosure.

**Claims**

1. A method for adjusting a steering of an endoscope, the endoscope comprising: a plurality of joints, a steering wire provided with a tension sensor, said steering wire passing through the plurality of joints, and a driver, one end of said steering wire being connected to said driver, the method comprising
a controller comprising a processor and memory communicably connected thereto performing the steps of

   acquiring a bending direction and a bending angle $\theta_1$ of the endoscope when steering of the endoscope is to be adjusted;
   determining, based on the bending direction, a steering steel wire to be adjusted among the plurality of steering steel wires, and determining, based on the bending angle $\theta_1$, a tension value $F_{y1}$ of the steering steel wire to be adjusted and a rotation angle of the driver corresponding to the steering steel wire to be adjusted;
   controlling the driver corresponding to the steering steel wire to be adjusted to rotate by the rotation angle;
   reading a value of the tension sensor corresponding to the steering steel wire to be adjusted, and recording the value as $F_1$;
   identifying whether $F_1$ is less than $F_{y1}$; and
   controlling the driver corresponding to the steering steel wire to be adjusted to stop working when an absolute value of a difference between $F_1$ and $F_y$ is less than a threshold, or continuing to control the driver corresponding to the steering steel wire to be adjusted to rotate in a same direction until the value of the tension sensor corresponding to the steering steel wire to be adjusted is equal to $F_{y1}$, if $F_1$ is less than $F_y$, **characterised in that** said endoscope comprises a plurality of drivers, an elastic member disposed between two adjacent joints of the joints and a plurality of steering wires, said wires being made of steel, whereby each of the steering steel wires are provided with a tension sensor with one end of each of said steering wires being connected to a different driver. ▪

2. The method according to claim 1, further comprising:

   during rotation of the driver corresponding to the steering steel wire, acquiring in real time, by the tension sensor on a steering steel wire to be adjusted, a real-time tension withstood by the steering steel wire to be adjusted;
   identifying whether the real-time tension is greater than or equal to a predetermined tension threshold;
   controlling the driver corresponding to the steering steel wire to be adjusted to stop working if the real-time tension is greater than or equal to the predetermined tension threshold; and
   proceeding with the step of reading the value of the tension sensor corresponding to the steering steel wire to be adjusted, and recording the value as $F_1$ after the rotation angle of the driver corresponding to the steering steel wire to be adjusted is reached, if the real-time tension is not greater than or not equal to a predetermined tension threshold.

3. The method according to claim 2, further comprising:
   outputting a prompt signal indicating that the endoscope fails to be bent to the bending angle when it is identified that the real-time tension is greater than or equal to the predetermined tension threshold.

4. The method according to claim 2, further comprising:

   acquiring the difference between $F_1$ and $F_{y1}$ when $F_1$ is less than $F_{y1}$;
   identifying whether the absolute value of the difference between $F_1$ and $F_{y1}$ is greater than or equal to a predetermined aging threshold;
   outputting a prompt signal indicating that the steering steel wire to be adjusted severely ages if the absolute value of the difference between $F_1$ and $F_{y1}$ is greater than or equal to the predetermined aging threshold; and
   proceeding with the step of continuing to control the driver corresponding to the steering steel wire to be adjusted to rotate in a same direction, if the absolute value of the difference between $F_1$ and $F_{y1}$ is less than the predetermined aging threshold.

5. The method according to any one of claims 1 to 4, wherein

   the bending direction of the endoscope is in correspondence with the steering steel wire; and
   a bended angle of the endoscope, the tension of the steering steel wire, and the rotation angle of the driver are in corresponding with each other.

**6.** An endoscope, comprising: a plurality of joints, a plurality of steering steel wires, and a plurality of drivers, and a controller, each of the steering steel wires passing through the plurality of joints, an elastic member being disposed between two adjacent joints of the joints, each of the steering steel wires being provided with a tension sensor, one end of each of the steering steel wires being connected to a driver; wherein

the controller comprises at least one processor and a memory communicably connected to the at least one processor; wherein
the memory stores instructions executable by the at least one processor, wherein, the instructions, when being executed by the at least one processor, cause the at least one processor to perform steps of:

acquiring a bending direction and a bending angle $\theta_1$ of the endoscope when steering of the endoscope is to be adjusted;
determining, based on the bending direction, a steering steel wire to be adjusted among the plurality of steering steel wires, and determining, based on the bending angle $\theta_1$, a tension value $F_{y1}$ of the steering steel wire to be adjusted and a rotation angle of the driver corresponding to the steering steel wire to be adjusted;
controlling the driver corresponding to the steering steel wire to be adjusted to rotate by the rotation angle;
reading a value of the tension sensor corresponding to the steering steel wire to be adjusted, and recording the value as $F_1$;
identifying whether $F_1$ is less than $F_{y1}$; and
controlling the driver corresponding to the steering steel wire to be adjusted to stop working when an absolute value of a difference between $F_1$ and $F_{y1}$ is less than a threshold, or continuing to control the driver corresponding to the steering steel wire to be adjusted to rotate in a same direction until the value of the tension sensor corresponding to the steering steel wire to be adjusted is equal to $F_{y1}$, if $F_1$ is less than $F_{y1}$.

**7.** The endoscope according to claim 6, wherein the instructions, when being executed by the at least one processor, cause the at least one processor to perform steps of:

during rotation of the driver corresponding to the steering steel wire, acquiring in real time, by the tension sensor on a steering steel wire to be adjusted, a real-time tension withstood by the steering steel wire to be adjusted;
identifying whether the real-time tension is greater than or equal to a predetermined tension threshold;
controlling the driver corresponding to the steering steel wire to be adjusted to stop working if the real-time tension is greater than or equal to the predetermined tension threshold; and
proceeding with the step of reading the value of the tension sensor corresponding to the steering steel wire to be adjusted, and recording the value as $F_1$ after the rotation angle of the driver corresponding to the steering steel wire to be adjusted is reached, if the real-time tension is not greater than or not equal to a predetermined tension threshold.

**8.** The endoscope according to claim 7, wherein the instructions, when being executed by the at least one processor, cause the at least one processor to perform a step of:
outputting a prompt signal indicating that the endoscope fails to be bent to the bending angle when it is identified that the real-time tension is greater than or equal to the predetermined tension threshold.

**9.** The endoscope according to claim 8, wherein the instructions, when being executed by the at least one processor, cause the at least one processor to perform steps of:

acquiring the difference between $F_1$ and $F_{y1}$ when $F_1$ is less than $F_{y1}$;
identifying whether the absolute value of the difference between $F_1$ and $F_{y1}$ is greater than or equal to a predetermined aging threshold;
outputting a prompt signal indicating that the steering steel wire to be adjusted severely ages if the absolute value of the difference between $F_1$ and $F_{y1}$ is greater than or equal to the predetermined aging threshold; and
proceeding with the step of continuing to control the driver corresponding to the steering steel wire to be adjusted to rotate in a same direction until the value of the tension sensor corresponding to the steering steel wire to be adjusted is equal to $F_{y1}$, if the absolute value of the difference between $F_1$ and $F_{y1}$ is less than the predetermined aging threshold.

**10.** The endoscope according to any one of claims 6 to 9, wherein

the bending direction of the endoscope is in correspondence with the steering steel wire; and
a bended angle of the endoscope, the tension of the steering steel wire, and the rotation angle of the driver are in corresponding with each other.

**11.** A controller, comprising:
at least one processor and a memory communicably connected to the at least one processor; wherein the memory stores instructions executable by the at least one processor, wherein, the instructions, when being executed by the at least one processor, cause the at least one processor to perform the method for adjusting a steering of an endoscope as defined in any one of claims 1 to 5.

**Patentansprüche**

**1.** Verfahren zum Justieren der Lenkung eines Endoskops, wobei das Endoskop umfasst: eine Vielzahl von Gelenken, einen Lenkungsdraht, der mit einem Spannungssensor versehen ist, wobei der Lenkungsdraht durch die Vielzahl von Gelenken verläuft, und einen Antrieb, wobei ein Ende des Lenkungsdrahts mit dem Antrieb verbunden ist, wobei das Verfahren umfasst, dass eine Steuereinrichtung, umfassend einen Prozessor und einen Speicher, der kommunizierend damit verbunden ist, folgende Schritte durchführt:

Erfassen einer Biegerichtung und eines Biegewinkels $\theta_1$ des Endoskops, wenn die Lenkung des Endoskops justiert werden soll;
Bestimmen, auf Grundlage der Biegerichtung, eines zu justierenden Lenkungsstahldrahts unter einer Vielzahl von Lenkungsstahldrähten, und Bestimmen, auf Grundlage des Biegewinkels $\theta_1$, eines Spannungswerts $F_{y1}$ des zu justierenden Lenkungsstahldrahts und eines Rotationswinkels des Antriebs, der dem zu justierenden Lenkungsstahldraht entspricht;
Steuern des Antriebs, der dem zu justierenden Lenkungsstahldraht entspricht, sodass er sich um den Rotationswinkel dreht;
Auslesen eines Werts des Spannungssensors, der dem zu justierenden Lenkungsstahldraht entspricht, und Aufzeichnen des Werts als $F_1$;
Ermitteln, ob $F_1$ kleiner als $F_{y1}$ ist; und
Steuern des Antriebs, der dem zu justierenden Lenkungsstahldraht entspricht, sodass er angehalten wird, wenn ein absoluter Wert einer Differenz zwischen $F_1$ und $F_{y1}$ kleiner als ein Schwellenwert ist, oder Steuern des Antriebs, der dem zu justierenden Lenkungsstahldraht entspricht, sodass er sich weiter in derselben Richtung dreht, bis der Wert des Spannungssensors, der dem zu justierenden Lenkungsstahldraht entspricht, gleich $F_{y1}$ ist, wenn $F_1$ kleiner als $F_{y1}$ ist, **dadurch gekennzeichnet, dass** das Endoskop eine Vielzahl von Antrieben, ein elastisches Element, das zwischen zwei benachbarten Gelenken der Gelenke angeordnet ist, und eine Vielzahl von Lenkungsdrähten umfasst, wobei die Drähte aus Stahl hergestellt sind, wobei jeder der Lenkungsstahldrähte mit einem Spannungssensor versehen ist, wobei ein Ende jedes Lenkungsdrahts mit einem anderen Antrieb verbunden ist.

**2.** Verfahren nach Anspruch 1, ferner umfassend:

während der Drehung des Antriebs, der dem Lenkungsstahldraht entspricht, Erlangen einer Echtzeitspannung, welcher ein zu justierender Lenkungsstahldraht standhalten kann, durch den Spannungssensor an dem zu justierenden Lenkungsstahldraht in Echtzeit;
Ermitteln, ob die Echtzeitspannung größer oder gleich einem vorgegebenen Spannungsschwellenwert ist;
Steuern des Antriebs, der dem zu justierenden Lenkungsstahldraht entspricht, sodass er angehalten wird, wenn die Echtzeitspannung größer oder gleich dem vorgegebenen Spannungsschwellenwert ist; und
Fortfahren mit dem Schritt des Auslesens des Werts des Spannungssensors, der dem zu justierenden Lenkungsstahldraht entspricht, und Aufzeichnen des Werts als $F_1$, nachdem der Rotationswinkel des Antriebs, der dem zu justierenden Lenkungsstahldraht entspricht, erreicht wurde, wenn die Echtzeitspannung größer oder gleich dem vorgegebenen Spannungsschwellenwert ist.

**3.** Verfahren nach Anspruch 2, ferner umfassend:
Ausgeben eines Aufforderungssignals, das angibt, dass das Endoskop nicht auf den Biegewinkel gebogen werden kann, wenn ermittelt wird, dass die Echtzeitspannung größer oder gleich dem vorgegebenen Spannungsschwellenwert ist.

4. Verfahren nach Anspruch 2, ferner umfassend:

Erlangen der Differenz zwischen $F_1$ und $F_{y1}$, wenn $F_1$ kleiner als $F_{y1}$ ist;
Ermitteln, ob der absolute Wert der Differenz zwischen $F_1$ und $F_{y1}$ größer oder gleich einem vorgegebenen Alterungsschwellenwert ist;
Ausgeben eines Aufforderungssignals, das angibt, dass der zu justierende Lenkungsstahldraht stark altert, wenn der absolute Wert der Differenz zwischen $F_1$ und $F_{y1}$ größer oder gleich dem vorgegebenen Alterungsschwellenwert ist; und
Übergehen zu dem Schritt des Steuerns des Antriebs, der dem zu justierenden Lenkungsstahldraht entspricht, sodass er sich weiter in derselben Richtung dreht, wenn der absolute Wert der Differenz zwischen $F_1$ und $F_{y1}$ kleiner als der vorgegebene Alterungsschwellenwert ist.

5. Verfahren nach einem der Ansprüche 1 bis 4, wobei

die Biegerichtung des Endoskops dem Lenkungsstahldraht entspricht; und
ein Biegewinkel des Endoskops, die Spannung des Lenkungsstahldrahts und der Rotationswinkel des Antriebs einander entsprechen.

6. Endoskop, umfassend: eine Vielzahl von Gelenken, eine Vielzahl von Lenkungsstahldrähten und eine Vielzahl von Antrieben und eine Steuereinrichtung, wobei jeder Lenkungsstahldraht durch die Vielzahl von Gelenken verläuft, ein elastisches Element zwischen zwei benachbarten Gelenken der Gelenke angeordnet ist, jeder Lenkungsstahldraht mit einem Spannungssensor versehen ist und ein Ende der Lenkungsstahldrähte mit einem Antrieb verbunden ist; wobei

die Steuereinrichtung wenigstens einen Prozessor und einen Speicher umfasst, der kommunizierend mit dem wenigstens einen Prozessor verbunden ist; wobei
der Speicher Anweisungen speichert, die durch den wenigstens einen Prozessor ausführbar sind, wobei die Anweisungen bei Ausführung durch den wenigstens einen Prozessor bewirken, dass der wenigstens eine Prozessor folgende Schritte durchführt:

Erfassen einer Biegerichtung und eines Biegewinkels $\theta 1$ des Endoskops, wenn die Lenkung des Endoskops justiert werden soll;
Bestimmen, auf Grundlage der Biegerichtung, eines zu justierenden Lenkungsstahldrahts unter der Vielzahl von Lenkungsstahldrähten, und Bestimmen, auf Grundlage des Biegewinkels $\theta 1$, eines Spannungswerts $F_{y1}$ des zu justierenden Lenkungsstahldrahts und eines Rotationswinkels des Antriebs, der dem zu justierenden Lenkungsstahldraht entspricht;
Steuern des Antriebs, der dem zu justierenden Lenkungsstahldraht entspricht, sodass er sich um den Rotationswinkel dreht;
Auslesen eines Werts des Spannungssensors, der dem zu justierenden Lenkungsstahldraht entspricht, und Aufzeichnen des Werts als $F_1$;
Ermitteln, ob $F_1$ kleiner als $F_{y1}$ ist; und
Steuern des Antriebs, der dem zu justierenden Lenkungsstahldraht entspricht, sodass er angehalten wird, wenn ein absoluter Wert einer Differenz zwischen $F_1$ und $F_{y1}$ kleiner als ein Schwellenwert ist, oder Steuern des Antriebs, der dem zu justierenden Lenkungsstahldraht entspricht, sodass er sich weiter in derselben Richtung dreht, bis der Wert des Spannungssensors, der dem zu justierenden Lenkungsstahldraht entspricht, gleich $F_{y1}$ ist, wenn $F_1$ kleiner als $F_{y1}$ ist.

7. Endoskop nach Anspruch 6, wobei die Anweisungen bei Ausführung durch den wenigstens einen Prozessor bewirken, dass der wenigstens eine Prozessor folgende Schritte durchführt:

während der Drehung des Antriebs, der dem Lenkungsstahldraht entspricht, Erlangen einer Echtzeitspannung, welcher ein zu justierender Lenkungsstahldraht standhalten kann, durch den Spannungssensor an dem zu justierenden Lenkungsstahldraht in Echtzeit;
Ermitteln, ob die Echtzeitspannung größer oder gleich einem vorgegebenen Spannungsschwellenwert ist;
Steuern des Antriebs, der dem zu justierenden Lenkungsstahldraht entspricht, sodass er angehalten wird, wenn die Echtzeitspannung größer oder gleich dem vorgegebenen Spannungsschwellenwert ist; und
Fortfahren mit dem Schritt des Auslesens des Werts des Spannungssensors, der dem zu justierenden Lenkungsstahldraht entspricht, und Aufzeichnen des Werts als $F_1$, nachdem der Rotationswinkel des Antriebs, der dem zu justierenden Lenkungsstahldraht entspricht, erreicht wurde, wenn die Echtzeitspannung größer oder gleich dem vorgegebenen Spannungsschwellenwert ist.

**8.** Endoskop nach Anspruch 7, wobei die Anweisungen bei Ausführung durch den wenigstens einen Prozessor bewirken, dass der wenigstens eine Prozessor folgenden Schritt durchführt:
Ausgeben eines Aufforderungssignals, das angibt, dass das Endoskop nicht auf den Biegewinkel gebogen werden konnte, wenn ermittelt wird, dass die Echtzeitspannung größer oder gleich dem vorgegebenen Spannungsschwellenwert ist.

**9.** Endoskop nach Anspruch 8, wobei die Anweisungen bei Ausführung durch den wenigstens einen Prozessor bewirken, dass der wenigstens eine Prozessor folgende Schritte durchführt:

Erlangen der Differenz zwischen $F_1$ und $F_{y1}$, wenn $F_1$ kleiner als $F_{y1}$ ist;
Ermitteln, ob der absolute Wert der Differenz zwischen $F_1$ und $F_{y1}$ größer oder gleich einem vorgegebenen Alterungsschwellenwert ist;
Ausgeben eines Aufforderungssignals, das angibt, dass der zu justierende Lenkungsstahldraht stark altert, wenn der absolute Wert der Differenz zwischen $F_1$ und $F_{y1}$ größer oder gleich dem vorgegebenen Alterungsschwellenwert ist; und
Übergehen zu dem Schritt des Steuerns des Antriebs, der dem zu justierenden Lenkungsstahldraht entspricht, sodass er sich weiter in derselben Richtung dreht, bis der Wert des Spannungssensors, der dem zu justierenden Lenkungsstahldraht entspricht, gleich $F_{y1}$ ist, wenn der absolute Wert der Differenz zwischen $F_1$ und $F_{y1}$ kleiner als der vorgegebene Alterungsschwellenwert ist.

**10.** Endoskop nach einem der Ansprüche 6 bis 9, wobei

die Biegerichtung des Endoskops dem Lenkungsstahldraht entspricht; und
ein Biegewinkel des Endoskops, die Spannung des Lenkungsstahldrahts und der Rotationswinkel des Antriebs einander entsprechen.

**11.** Steuereinrichtung, umfassend:
wenigstens einen Prozessor und einen Speicher, der kommunizierend mit dem wenigstens einen Prozessor verbunden ist; wobei der Speicher Anweisungen speichert, die durch den wenigstens einen Prozessor ausführbar sind, wobei die Anweisungen bei Ausführung durch den wenigstens einen Prozessor bewirken, dass der wenigstens eine Prozessor das Verfahren zum Justieren der Lenkung eines Endoskops nach einem der Ansprüche 1 bis 5 durchführt.

**Revendications**

**1.** Un procédé pour ajuster le guidage d'un endoscope, l'endoscope comprenant: une pluralité de joints, un câble de guidage muni d'un capteur de tension, ledit câble de guidage passant à travers la pluralité de joints, et un pilote, une extrémité dudit câble de guidage étant connectée audit pilote, le procédé comprenant un contrôleur comprenant un processeur et une mémoire qui y est connectée de façon communiquante et effectuant les étapes d'acquisition d'une direction de flexion et d'un angle de flexion $\theta_1$ de l'endoscope lorsque le guidage de l'endoscope doit être ajusté ;

déterminant, en se basant sur la direction de flexion, un câble de guidage en acier à ajuster parmi la pluralité de câbles de guidage en acier, et déterminant, en se basant sur angle de flexion $\theta_1$, une valeur de tension $Fy_1$ du câble de guidage en acier à ajuster et un angle de rotation du pilote correspondant au câble de guidage en acier à ajuster ;
contrôlant la rotation du pilote correspondant au câble de guidage en acier à ajuster afin qu'il pivote selon l'angle de rotation ;
lisant une valeur du capteur de tension correspondant au câble de guidage en acier à ajuster, et enregistrant la valeur comme $F_1$;
identifiant si $F_1$ est inférieure à $Fy_1$; et
contrôlant l'arrêt du fonctionnement du pilote correspondant au câble de guidage en acier à ajuster lorsqu'une valeur absolue de la différence entre $F_1$ et $Fy_1$ est inférieure à un seuil, ou continuant de contrôler la rotation du pilote correspondant au câble de guidage en acier à ajuster dans la même direction jusqu'à ce que la valeur du capteur de tension correspondant au câble de guidage en acier à ajuster soit égale à $Fy_1$, si $F_1$ est inférieure à $Fy_1$, **caractérisé en ce que** ledit endoscope comprend une pluralité de pilotes, un élément élastique agencé entre deux joints adjacents parmi les joints et une pluralité de câbles de guidage, lesdits câbles de guidage étant en acier, dans lesquels chacun des câbles de guidage en acier est muni d'un capteur de tension et une extrémité de chacun desdits câbles en acier étant connectée à un pilote différent.

2. Un procédé selon la revendication 1, comprenant en outre :

durant la rotation du pilote correspondant au câble de guidage en acier, l'acquisition en temps réel, par le capteur de tension sur le câble de guidage en acier à ajuster, de la tension en temps réel supportée par le câble de guidage en acier à ajuster ;
identifier si la tension en temps réel est supérieure ou égale à un seuil de tension prédéterminé ;
contrôler l'arrêt du pilote correspondant au câble de guidage en acier à ajuster si la tension en temps réel est supérieure au seuil de tension prédéterminé ; et
passer à l'étape de lecture de la valeur du capteur de tension correspondant au câble de guidage en acier à ajuster, et enregistrer la valeur en tant que $F_1$ une fois que l'angle de rotation du pilote correspondant au câble de guidage en acier à ajuster est atteinte, si la tension en temps réel n'est pas supérieure ou égale à un seuil de tension prédéterminé.

3. Un procédé selon la revendication 2, comprenant en outre :
l'émission d'un signal immédiat indiquant que l'endoscope ne parvient pas à être fléchi à l'angle de flexion lorsqu'il est identifié que la tension en temps réel est supérieure ou égale à un seuil de tension prédéterminé.

4. Un procédé selon la revendication 2, comprenant en outre :

acquérir la différence entre $F_1$ et $Fy_1$ lorsque $F_1$ est inférieure à $Fy_1$ ;
identifier si la valeur absolue de la différence entre $F_1$ et $Fy_1$ est supérieure ou égale à un seuil de vieillissement prédéterminé ;
émettre un signal immédiat indiquant que le câble de guidage en acier doit être ajusté fortement en âge si la valeur absolue de la différence entre $F_1$ et $Fy_1$ est supérieure ou égale au seuil de vieillissement prédéterminé ; et
passer à l'étape de continuation du contrôle de rotation du pilote correspondant au câble de guidage en acier à ajuster dans la même direction, si la valeur absolue de la différence entre $F_1$ et $Fy_1$ est inférieure au seuil de vieillissement prédéterminé.

5. Un procédé selon l'une quelconque des revendications 1 à 4, dans lequel

la direction de flexion de l'endoscope est en correspondance avec le câble de guidage en acier ; et
un angle de flexion de l'endoscope, la tension du câble de guidage en acier, et l'angle de rotation of pilote correspondent mutuellement.

6. Un endoscope, comprenant : une pluralité de joints, une pluralité de câbles de guidage en acier, et une pluralité de pilotes, et un contrôleur, chaque câble de guidage en acier passant à travers la pluralité de joints, un élément élastique agencé entre deux joints adjacents parmi les joints, chaque câble de guidage en acier étant muni d'un capteur de tension, une extrémité de chacun desdits câbles de guidage en acier étant connectée à un pilote ; dans lequel

le contrôleur comprend au moins un processeur et une mémoire connectée de façon communiquante au au moins un processeur ; dans lequel
la mémoire sauvegarde les instructions exécutables par le au moins un processeur, dans lequel, les instructions, lorsque exécutées par le au moins un processeur, engendrent la performance par le au moins un processeur des étapes de :

acquisition d'une direction de flexion et d'un angle de flexion $\theta_1$ de l'endoscope lorsque le guidage de l'endoscope doit être ajusté ;
déterminer, en se basant sur la direction de flexion, un câble de guidage en acier à ajuster parmi la pluralité de câbles de guidage en acier, et déterminer, en se basant sur l'angle de flexion $\theta_1$, une valeur de tension $Fy_1$ du câble de guidage en acier à ajuster et un angle de rotation du pilote correspondant au câble de guidage en acier à ajuster ;
contrôler la rotation du pilote correspondant au câble de guidage en acier à ajuster afin qu'il pivote selon l'angle de rotation ;

lire une valeur du capteur de tension correspondant au câble de guidage en acier à ajuster, et enregistrer la valeur comme $F_1$ ;

identifier si $F_1$ est inférieure à $Fy_1$ ; et

contrôler l'arrêt du fonctionnement du pilote correspondant au câble de guidage en acier à ajuster lorsqu'une valeur absolue d'une différence entre $F_1$ et $Fy_1$ est inférieure à un seuil, ou continuer de contrôler la rotation du pilote correspondant au câble de guidage en acier à ajuster dans la même direction jusqu'à ce que la valeur du capteur de tension correspondant au câble de guidage en acier à ajuster soit égale à $Fy_1$, si $F_1$ est inférieure à $Fy_1$.

7. L'endoscope selon la revendication 6, dans lequel les instructions, lorsque exécutées par le au moins un processeur, engendrent la performance par le au moins un processeur des étapes de :

durant la rotation du pilote correspondant au câble de guidage en acier, l'acquisition en temps réel, par le capteur de tension sur le câble de guidage en acier à ajuster, de la tension en temps réel supportée par le câble de guidage en acier à ajuster ;

identifier si la tension en temps réel est supérieure ou égale à un seuil de tension prédéterminé ;

contrôler l'arrêt du pilote correspondant au câble de guidage en acier à ajuster si la tension en temps réel est supérieure au seuil de tension prédéterminé ; et

passer à l'étape de lecture de la valeur du capteur de tension correspondant au câble de guidage en acier à ajuster, et enregistrer la valeur en tant que $F_1$ une fois que l'angle de rotation du pilote correspondant au câble de guidage en acier à ajuster est atteinte, si la tension en temps réel n'est pas supérieure ou égale à un seuil de tension prédéterminé.

8. L'endoscope selon la revendication 7, dans lequel les instructions, lorsque exécutées par le au moins un processeur, engendrent la performance par le au moins un processeur d'une étape de :

émission d'un signal immédiat indiquant que l'endoscope ne parvient pas à être fléchi à l'angle de flexion lorsqu'il est identifié que la tension en temps réel est supérieure ou égale à un seuil de tension prédéterminé.

9. L'endoscope selon la revendication 8, dans lequel les instructions, lorsque exécutées par le au moins un processeur, engendrent la performance par le au moins un processeur des étapes de :

acquisition de la différence entre $F_1$ et $Fy_1$ lorsque $F_1$ est inférieure à $Fy_1$ ;

identifier si la valeur absolue de la différence entre $F_1$ et $Fy_1$ est supérieure ou égale à un seuil de vieillissement prédéterminé ;

émettre un signal immédiat indiquant que le câble de guidage en acier doit être ajusté fortement en âge si la valeur absolue de la différence entre $F_1$ et $Fy_1$ est supérieure ou égale au seuil de vieillissement prédéterminé ; et

passer à l'étape de continuation du contrôle de rotation du pilote correspondant au câble de guidage en acier à ajuster dans la même direction jusqu'à ce que la valeur du capteur de tension correspondant au câble de guidage en acier à ajuster soit égale à $Fy_1$, si la valeur absolue de la différence entre $F_1$ et $Fy_1$ est inférieure au seuil de vieillissement prédéterminé.

10. Un endoscope selon l'une quelconque des revendications 6 à 9, dans lequel

la direction de flexion de l'endoscope est en correspondance avec le câble de guidage en acier ; et

un angle de flexion de l'endoscope, la tension du câble de guidage en acier, et l'angle de rotation of pilote correspondent mutuellement.

11. Un contrôleur, comprenant :

au moins un processeur et une mémoire qui est connectée de façon communiquante au au moins un processeur ;

dans lequel la mémoire sauvegarde les instructions exécutables par le au moins un processeur, dans lequel, les instructions, lorsque exécutées par le au moins un processeur, engendrent la performance par le au moins un processeur du procédé pour ajuster le guidage d'un endoscope tel que défini dans l'une quelconque des revendications 1 à 5.

FIG. 1

FIG. 2

Acquire a bending direction and a bending angle θ1 of the endoscope when steering of the endoscope is to be adjusted ⌐ 31

Determine a steering steel wire to be adjusted among the plurality of steering steel wires based on the bending direction, and determine a tension value $F_{y1}$ of the steering steel wire to be adjusted and a rotation angle of the driver corresponding to the steering steel wire to be adjusted based on the bending angle θ1 ⌐ 32

Control the driver corresponding to the steering steel wire to be adjusted to rotate by the rotation angle ⌐ 33

Read a value of the tension sensor corresponding to the steering steel wire to be adjusted, and record the value as $F_1$ ⌐ 34

35

Identify whether $F_1$ is less than $F_{y1}$

Yes

No

Continue controlling the driver corresponding to the steering steel wire to be adjusted to rotate in the same direction continues until the value of the tension sensor corresponding to the steering steel wire to be adjusted is equal to $F_{y1}$

36

Control the driver corresponding to the steering steel wire to be adjusted to stop rotating

37

FIG. 3

Acquire a bending direction and a bending angle $\theta_1$ of the endoscope when steering of the endoscope is to be adjusted ⟍ 31

Determine, based on the bending direction, a steering steel wire to be adjusted among the plurality of steering steel wires, and determine, based on the bending angle $\theta_1$, a tension value $F_{y1}$ of the steering steel wire to be adjusted and a rotation angle of the driver corresponding to the steering steel wire to be adjusted ⟍ 32

Control the driver corresponding to the steering steel wire to be adjusted to rotate by the rotation angle ⟍ 33

During rotation of the driver corresponding to the steering steel wire, acquire a real-time tension withstood by the steering steel wire to be adjusted in real time by the tension sensor on a steering steel wire to be adjusted ⟍ 41

Identify whether the real-time tension is greater than or equal to a predetermined tension threshold

Yes ◄ | No ► 45

42

Control the driver corresponding to the steering steel wire to be adjusted to stop working ⟍ 43

Proceed with the step of reading the value of the tension sensor corresponding to the steering steel wire to be adjusted, and recording the value as $F_1$ after the rotation angle of the driver corresponding to the steering steel wire to be adjusted is reached

35

Output a prompt signal indicating that the endoscope fails to be bent to the bending angle when it is identified that the real-time tension is greater than or equal to the predetermined tension threshold ⟍ 44

36 ⟍ Yes | Identify whether $F_1$ is less than $F_{y1}$ | No

Continue controlling the driver corresponding to the steering steel wire to be adjusted to rotate in the same direction continues until the value of the tension sensor corresponding to the steering steel wire to be adjusted is equal to $F_{y1}$

37 ⟍ Control the driver corresponding to the steering steel wire to be adjusted to stop rotating

FIG. 4

Acquire a bending direction and a bending angle $\theta_1$ of the endoscope when steering of the endoscope is to be adjusted
31

Determine, based on the bending direction, a steering steel wire to be adjusted among the plurality of steering steel wires, and determine, based on the bending angle $\theta_1$, a tension value $F_{y1}$ of the steering steel wire to be adjusted and a rotation angle of the driver corresponding to the steering steel wire to be adjusted
32

Control the driver corresponding to the steering steel wire to be adjusted to rotate by the rotation angle
33

Read a value of the tension sensor corresponding to the steering steel wire to be adjusted, and record the value as $F_1$
34

35
Identify whether $F_1$ is less than $F_{y1}$ — No → Control the driver corresponding to the steering steel wire to be adjusted to stop rotating
37

Yes

Acquire a difference between $F_1$ and $F_{y1}$
51

54
Proceed with the step of continuing to control the driver corresponding to the steering steel wire to be adjusted to rotate in the same direction ← No — Identify whether the absolute value of the difference between $F_1$ and $F_{y1}$ is greater than or equal to a predetermined aging threshold — No → Output a prompt signal indicating that the steering steel wire to be adjusted severely ages
53

52

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1464270 A